# EUROPÄISCHE PATENTSCHRIFT

(11) **EP 0 930 875 B1**
(45) Veröffentlichungstag und Bekanntmachung des Hinweises auf die Patenterteilung: **07.05.2003**
(21) Anmeldenummer: 97942022.1
(22) Anmeldetag: 11.09.1997
(51) Int. Cl.: A61K 9/20, A61K 9/22

(54) **VERFAHREN ZUR HERSTELLUNG FESTER PHARMAZEUTISCHER FORMEN DURCH EXTRUDIERUNG**
PROCESS FOR PRODUCING SOLID DOSAGE FORMS BY EXTRUSION
PROCEDE DE FABRICATION DE FORMES PHARMACEUTIQUES SOLIDES PAR EXTRUSION

(30) Priorität: 13.09.1996 DE 19637479; 06.08.1997 DE 19734011
(43) Veröffentlichungstag der Anmeldung: 28.07.1999
(73) Patentinhaber: BASF AKTIENGESELLSCHAFT, 67056 Ludwigshafen (DE)
(72) Erfinder: BREITENBACH, Jörg, D-68199 Mannheim (DE); KLEINKE, Andreas, D-67063 Ludwigshafen (DE); KOTHRADE, Stephan, D-67117 Limburgerhof (DE); ROSENBERG, Joerg, D-67158 Ellerstadt (DE)
(74) Vertreter: Kinzebach, Werner, Dr.
(86) Internationale Anmeldenummer: EP9704984
(87) Internationale Veröffentlichungsnummer: WO98010752

(56) Entgegenhaltungen:
- EP-A- 0 080 862
- EP-A- 0 544 144
- EP-A- 0 729 748
- WO-A-92/05774
- FR-A- 2 179 044
- GB-A- 2 249 957
- US-A- 5 260 074
- "LOW TEMPERATURE MELT PROCESSING OF PHARMACEUTICAL SUBSTANCES IN POLY(ETHYLENE OXIDE) POLYMERS USING A TRANSIENT PLASTICIZER" RESEARCH DISCLOSURE, Nr. 387, Juli 1996, Seite 404 XP000599807
- PATENT ABSTRACTS OF JAPAN vol. 013, no. 015 (C-559), 13.Januar 1989 & JP 63 222112 A (NIPPON SODA CO LTD), 16.September 1988, & DATABASE WPI Section Ch, Week 8843 Derwent Publications Ltd., London, GB; Class A12, AN 88-303255 & JP 63 222 112 (NIPPON SODA) , 16.September 1988

## Beschreibung

Die vorliegende Erfindung betrifft ein Verfahren zur Herstellung fester pharmazeutischer Formen durch Vermischen von wenigstens einem pharmakologisch akzeptablen polymeren Bindemittel, wenigstens einem pharmazeutischen Wirkstoff und ggf. üblichen pharmazeutischen Additiven und Extrudieren des Gemisches zur gewünschten pharmazeutischen Form.

Die klassischen Verfahren zur Herstellung fester pharmazeutischer Formen, insbesondere Tabletten, werden diskontinuierlich durchgeführt und umfassen mehrere Stufen. Im Allgemeinen werden die Bestandteile der Arzneiform zunächst in einen geeigneten Behälter gefördert und dort unter Zusatz eines Lösungsmittels zu einem knetfähigen Teig vermischt. Anschließend granuliert man den Teig, trocknet das Granulat und formt es zu der gewünschten Arzneiform, beispielsweise durch Verpressen zu Tabletten. Derartige Verfahren sind in einschlägigen Lehrbüchern und beispielsweise in DE-A-41 41 268 und EP-A-590 963 beschrieben. Ein wesentlicher Nachteil dieser Verfahren liegt in der Vielzahl der erforderlichen Stufen und Apparaturen.

Seit einiger Zeit ist ein wesentlich einfacheres kontinuierliches Verfahren zur Herstellung fester pharmazeutischer Formen bekannt, bei dem man eine wirkstoffhaltige lösungsmittelfreie Schmelze aus einem polymeren Bindemittel durch Spritzgießen oder Extrudieren und anschließende Formgebung in die gewünschte Arzneiform überführt, siehe beispielsweise EP-A-240 904, EP-A-240 906 und EP-A-337 256. Ein solches Verfahren umfasst ein energieintensives Aufschmelzen des meist pulverförmigen thermoplastischen Bindemittels. Darüberhinaus erfordert das Vermischen des Bindemittels mit weiteren Komponenten, wie einem pharmazeutischen Wirkstoff oder üblichen galenischen Hilfsmitteln, die in der Regel in fester Form vorliegen, einen relativ hohen technischen Aufwand. So werden beispielsweise spezielle Feststoffmischer benötigt, falls das Vermischen in einem getrennten Verfahrensschritt vor dem Aufschmelzen erfolgen soll. In diesem Fall besteht darüberhinaus die Gefahr, dass sich die Komponenten der in den Extruder eingespeisten Vormischung entmischen und damit Arzneimittel ungleichmäßiger Zusammensetzung hergestellt werden. Dagegen erfordert die Kopplung des Aufschmelz- und Mischvorgangs im Extruder eine relativ lange Verweilzeit in einer Zone mit hoher Scherung, um eine ausreichende Vermischung der Komponenten zu bewirken. Dadurch kann es zu einer lokalen Überhitzung und Schädigung des Produktes, insbesondere bei Verwendung eines scher- und temperaturempfindlichen Wirkstoffes kommen. Ein weiterer Nachteil des Einsatzes pulverförmiger Stoffe besteht in der Staubbildung.

Es ist zwar richtig, dass der Transport trockener pulverförmiger Polymerisate kostengünstiger ist. Allerdings werden eine Vielzahl von pharmakologisch geeigneten, zur Herstellung von festen pharmazeutischen Formen als Bindemittel zur Anwendung kommenden Polymerisate als Dispersion oder Lösung hergestellt. Zur Verwendung in dem oben beschriebenen Verfahren werden die Polymerisate in einem weiteren Verfahrensschritt stets als Feststoff isoliert und getrocknet.

Polymerdispersionen werden bei der Herstellung fester pharmazeutischer Formen zum Aufbringen eines Überzugs eingesetzt. So beschreibt die EP 088 951 ein Verfahren zum Überziehen von festen Arzneiformen mittels eines in Wasser dispergierten Überzugsmittels. Dazu wird ein Emulsionspolymerisat sprühgetrocknet, redispergiert und die erhaltene Dispersion als Überzugsmittel auf die schon vorgefertigte Arzneiform aufgebracht.

Die EP-A-80862 beschreibt wasserdispergierbare Mittel, die ein nicht-hygroskopisches, wasserlösliches, polymeres Bindemittel umfassen. Dieses ist in einem pharmazeutisch verträglichen, organischen Lösungsmittel löslich. Das Lösungsmittel wird erst aus dem Extrudat entfernt.

Die US-A-5,260,074 beschreibt ein klassisches Granulierungsverfahren, wobei man eine Pulvermischung befeuchtet, granuliert und abschließend trocknet.

Die EP-A-544144 beschreibt ein Schmelzextrusionsverfahren, bei dem eine Mischung des pharmazeutischen Wirkstoffs mit polymeren Bindemitteln und gegebenenfalls weiteren üblichen Zusätzen lösungsmittelfrei, d. h. ohne Wasser und ohne organische Lösungsmittel hergestellt und extrudiert wird.

Die FR-A-2179044 beschreibt eine Dosierungsform für chemisch aktive Verbindungen, die aus einem Träger aus einer makromolekularen Gelgerüstmatrix aufgebaut ist. In den Zwischenplätzen der Gelgerüstmatrix sind 4 bis 50 Gew.-% Wasser enthalten. Beim Verarbeiten mittels Extrusion oder Spritzguss enthält der Träger 15 bis 20 Gew.-% Wasser. Die Herstellung der Gelgerüstmatrix erfolgt durch Zugabe von Wasser und gegebenenfalls Weichmachern und anderen Additiven unter Rühren, wobei ein Granulat gebildet wird.

In der Research Disclosure, July 1996, S. 404 wird ein Schmelzextrusionsverfahren zur Herstellung pharmazeutischer Dosierungsformen beschrieben, wobei als Bindemittel ein hochmolekulares Poly(ethylenoxid) verwendet wird. Das Polyethylenoxid wird mit Wasser als Weichmacher vor dem Vermischen mit den anderen Bestandteilen hydratisiert. Bei der Hydratisierung quillt das Polyethylenglykol.

Die EP-A-0729748 beschreibt ein Verfahren zur Herstellung einer pharmazeutischen Zusammensetzung durch Extrusion eines Gemisches aus einem physiologisch inerten, pulverförmigen Additiv, einem makromolekularen Additiv und wenigstens einem Wirkstoff. Das Lösungsmittel wird während der Extrusion nicht verdampft, da das Extrudat abschließend einem Trocknungsschritt unterzogen wird.

Die WO 92/05774 beschreibt eine lösungsmittelfreie, oral zu verabreichende pharmazeutische Zubereitung, die durch Schmelzextrusion hergestellt werden kann. Der geschmolzene Wirkstoff dient als Lösungsmittel für mindestens einen zu lösenden, die Retardierung bewirkenden Hilfsstoff.

Die JP-A-63222112 (WPI Abstract, PAJ Abstract) beschreibt ein klassischen Granulierverfahren, bei dem zunächst die drei Komponenten vermischt werden und anschließend Ethanol hinzugefügt wird. Im Anschluss daran erfolgt Extrudieren und Granulieren.

Die GB-A-2249957 beschreibt extrudierte Zusammensetzungen, die eine kontrollierte Freisetzung des Wirkstoffs ermöglichen. Die Zusammensetzungen umfassen auch ein wasserlösliches polymeres Bindemittel, das in Form einer wässrigen Lösung eingesetzt wird. Das Gemisch der Komponenten wird extrudiert, ein Verdampfen des Wassers bei der Extrusion erfolgt jedoch nicht.

Gegenstand der vorliegenden Erfindung ist ein Verfahren zur Herstellung fester Dosierungsformen durch Vermischen von 50 bis 99,9 Gew.-% wenigstens eines polymeren Bindemittels, das ausgewählt ist unter Poly(meth)acrylaten, deren Copolymerisaten mit (Meth)acrylsäure, Polyvinyllactamen, Polyvinylestern und Copolymerisaten aus N-Vinyllactamen und Vinylestern, 0,1 bis 50 Gew.-% (jeweils bezogen auf das Gesamtgewicht der Dosierungsform) wenigstens eines Wirkstoffs und gegebenenfalls üblichen Additiven, Extrudieren des Gemisches, wobei man zumindest einen Teil des polymeren Bindemittels in Form einer wässrigen Dispersion einsetzt und das Dispersionsmittel im Extruder verdampft, und Formgebung.

Unter Dosierungsformen sind hier alle Formen zu verstehen, die zur Verwendung als Arzneimittel, Pflanzenbehandlungsmittel, Futtermittel und Nahrungsmittel geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Als Bindemittel eignen sich insbesondere pharmakologisch akzeptable Polymerisate. Darunter versteht man physiologisch verträgliche Polymerisate, die in physiologischer Umgebung löslich oder quellbar sind und den enthaltenen Wirkstoff freisetzen. Des weiteren ist von Bedeutung, dass die Verarbeitungstemperatur so gewählt werden kann, dass einerseits das Gemisch formbar ist, andererseits aber keine Schädigung der Komponenten, insbesondere des Wirkstoffes, eintritt. Das bedingt, dass die Glasübergangstemperatur des polymeren Bindemittels vorzugsweise unter der Zersetzungstemperatur aller in dem Gemisch enthaltenen Komponenten liegt.

Erfindungsgemäß geeignete Polymerdispersionen oder -lösungen können durch Emulsions-, Suspensions- bzw. Lösungspolymerisation erhalten werden. Diese Verfahren sind dem Fachmann bekannt. Gegebenenfalls benötigte Hilfsstoffe, die zur Durchführung dieser Verfahren eingesetzt werden, sind ebenfalls Bestandteil des erfindungsgemäßen Bindemittels und werden weiter unten im Detail beschrieben.

Des weiteren kann man erfindungsgemäß geeignete Polymerdispersionen oder -lösungen durch Dispergieren bzw. Lösen eines festen Polymerisats in einem Dispergier- oder Lösungsmittel erhalten. Beispielsweise kann man ein Polymerisat in einem organischen Lösungsmittel lösen und anschließend in die erhaltene organische Lösung sukzessive eine wässrige Lösung mit Emulgatoren, die geeignet sind, das Polymerisat in Wasser zu dispergieren, einrühren. Dabei entsteht zunächst - solange die organische Phase im Überschuss vorhanden ist - eine W/O-Emulsion. Bei weiterem Zusatz der Emulgatorlösung findet schließlich eine Phaseninversion statt, wobei sich eine feindisperse O/W-Emulsion bildet. Daraus kann durch Abdampfen der organischen Lösungsmittel eine Dispersion, vorzugsweise eine wässrige Dispersion, gewonnen werden. Hilfsstoffe, die ggf. zur Durchführung dieses Verfahrens zur Anwendung kommen, sind Bestandteil der erfindungsgemäß geeigneten Dispersionen oder Lösungen und werden weiter unten im Detail beschrieben. Vorzugsweise kommen jedoch die bei der Herstellung des Polymerisats erhaltenen Lösungen oder Dispersionen, ggf. nach Konzentrierung, zur Anwendung.

In dem erfindungsgemäßen Verfahren können sowohl W/O-Emulsionen als auch O/W-Emulsionen eingesetzt werden.

Die erfindungsgemäß geeigneten Polymerisate sind durch Polymerisation von ethylenisch ungesättigten Monomeran erhältlich. Es handelt sich dabei sowohl um im Wesentlichen wasserunlösliche wie auch im Wesentlichen wasserlösliche Monomere. Sie können alleine oder im Gemisch eingesetzt werden. Der relative Anteil von wasserunlöslichen bzw. wasserlöslichen Monomeren beeinflusst in erheblichem Maß die Eigenschaften des resultierenden Polymerisates. Ort und Zeit der Wirkstofffreisetzung lassen sich daher den galenischen Anforderungen entsprechend durch die Wahl des Polymerisats einstellen.

Geeignete, im Wesentlichen wasserunlösliche Monomere sind insbesondere monoethylenisch ungesättigte Monomere, wie Ester aus Acrylsäure oder Methacrylsäure, mit im Allgemeinen 1 bis 30, vorzugsweise 1 bis 18 und insbesondere 1 bis 12 C-Atome aufweisenden Alkanolen, und Vinylester von aliphatischen C₁-C₁₈-Monocarbonsäuren. Beispiele sind Methylmethacrylat, Ethylacrylat, Ethylmethacrylat, n-Propylacrylat, n-Propylmethacrylat, Isopropylacrylat, Isopropylmethacrylat, n-Butylacrylat, n-Butylmethacrylat, Isobutylacrylat, Isobutylmethacrylat, t-Butylacrylat, t-Butylmethacrylat, Pentylacrylat, Pentylmethacrylat, n-Hexylacrylat, n-Hexylmethacrylat, n-Heptylacrylat, n-Heptylmethacrylat, n-Octylacrylat. n-Octylmethacrylat, 2-Ethylhexylacrylat, 2-Ethylhexylmethacrylat, Decylacrylat, Decylmethacrylat, Laurylacrylat, Laurylmethacrylat, Palmitylacrylat, Palmitylmethacrylat, Stearylacrylat, Stearylmethacrylat, Hydrenolacrylat, Hydrenolmethacrylat, Behenylacrylat oder Behenylmethacrylat, Vinylacetat, Vinylpropionat, Vinyl-n-butyrat, Vinyllaurat, Virylstearat.

Wasserlösliche Monomere sind die α,β-monoethylenisch ungesättigten Carbonsäuren Acrylsäure und Methacrylsäure; N-Vinyllactame, wie N-Vinylpyrrolidon oder N-Vinylcaprolactam.

Besonders bevorzugt sind einerseits Poly(meth)acrylate und deren Copolymerisate mit (Meth)acrylsäure, insbesondere ein Copolymerisat aus Methacrylsäure und Ethylacrylat, andererseits Polyvinylpyrrolidon und dessen Copolymerisat mit Vinylestern, insbesondere ein Copolymerisat aus N-Vinylpyrrolidon und Vinylacetat.

Die K-Werte (nach H. Fikentscher, Cellulose-Chemie 13 (1932), Seiten 58 bis 64 und 71 und 74) der Polymere liegen im Bereich von 10 bis 100, vorzugsweise 12 bis 70, insbesondere 12 bis 35, für PVP>17, insbesondere 20 bis 35.

Vorzugsweise werden die vorstehend beschriebenen Polymerisate durch radikalische, wässrige Emulsionspolymerisation hergestellt, so dass sie als wässrige Dispersion erhalten werden. Besonders bevorzugt sind Latex-Dispersionen, unter denen man in der makromolekularen Chemie Dispersionen versteht, die halbfeste, plastische oder elastische Partikel sehr fein dispergiert enthalten. Derartige Latexpartikel weisen eine nahezu kugelförmige Gestalt mit einem Durchmesser von 0,01 bis 1 µm auf.

Die erfindungsgemäß geeigneten Polymerisate können auch weitere Hilfsstoffe umfassen, die bei der Herstellung dieser Polymerisate eine Rolle spielen. Dazu zählen beispielsweise die radikalischen Polymerisationsinitiatoren, wie beispielsweise Peroxide, Hydroperoxide, Peroxidisulfate, Percarbonate, Peroxiester und Azoverbindungen. Sie zersetzen sich unter den Extrudierbedingungen oder sie werden nach der Polymerisation durch eine geeignete Nachbehandlung mit Reduktionsmitteln zersetzt.

Polymerdispersionen und -lösungen in einem wässrigen Medium werden erfindungsgemäß bevorzugt. Unter wässrigem Medium sind hier auch Mischungen aus Wasser und damit mischbaren organischen Flüssigkeiten zu verstehen. Mit Wasser mischbare organische Flüssigkeiten sind beispielsweise Polyole, insbesondere Glykole wie Ethylenglykol, Propylenglykol, 1,3-Butylenglykol, Diethylenglykol, Triethylenglykol, Tetraethylenglykol und Glycerin, Blockcopolymerisate aus Ethylenoxid und Propylenoxid, alkoxylierte C₁ - C₂₀-Alkohole, Essigsäureester von Glykolen und Polyglykolen, Alkohole wie Methanol, Ethanol, Isopropanol und Butanol, Aceton, Tetrahydrofuran, Dimethylformamid, N-Methylpyrrolidon oder auch Mischungen der genannten Lösemittel. Falls die Polymerisation in Mischungen aus Wasser und mit Wasser mischbaren Lösemitteln erfolgt, so beträgt der Anteil an mit Wasser mischbaren Lösemitteln in der Mischung bis zu 75 Gew.-%.

Zur Herstellung der erfindungsgemäß geeigneten Dispersionen kommen bevorzugt physiologisch verträgliche Emulgatoren oder Schutzkolloide als Dispergiermittel zur Anwendung. Dies gilt sowohl für die Durchführung der radikalischen Polymerisation, insbesondere Emulsionspolymerisation, als auch für die Redispergierung lösungsmittelfreier Polymerisate.

Geeignete Schutzkolloide sind beispielsweise Polyvinylalkohole, Cellulosederivate, Polyvinylpyrrolidon oder Vinylpyrrolidon enthaltende Copolymerisate. Eine ausführliche Beschreibung weiterer geeigneter Schutzkolloide findet sich in Houben-Weyl, Methoden der organischen Chemie, Band XIV/1, Makromolekulare Stoffe, Georg-Thieme-Verlag, Stuttgart, 1961, S. 411 bis 420. Selbstverständlich können auch Gemische aus Emulgatoren und/oder Schutzkolloiden verwendet werden. Vorzugsweise werden als Dispergiermittel ausschließlich Emulgatoren eingesetzt, deren relative Molekulargewichte im Unterschied zu den Schutzkolloiden üblicherweise unter 1000 liegen. Sie können sowohl anionischer, kationischer oder nichtionischer Natur sein. Gebräuchliche Emulgatoren sind z. B. ethoxylierte Mono-, Di- und Tri-Alkylphenole (EO-Grad: 3 bis 50, Alkylrest: C₄ bis C₉), ethoxylierte Fettalkohole (EO-Grad: 3 bis 50, Alkylrest: C₈ bis C₃₆), sowie Alkali- und Ammoniumsalze von Alkylsulfaten (Alkylrest: C₈ bis C₁₂), von Schwefelsäurehalbestern ethoxylierter Alkanole (EO-Grad: 4 bis 30, Alkylrest: C₁₂ bis C₁₈) und ethoxylierter Alkylphenole (EO-Grad: 3 bis 50, Alkylrest: C₄ bis C₉), von Alkylsulfonsäuren (Alkylrest: C₁₂ bis C₁₈) und von Alkylarylsulfonsäuren (Alkylrest: C₉ bis C₁₈). Weitere geeignete Emulgatoren finden sich in Houben-Weyl, Methoden der organischen Chemie, Band XIV/1, Makromolekulare Stoffe, Georg-Thieme Verlag, Stuttgart, 1961, Seiten 192 bis 208).

Weiterhin können den Bindemitteln übliche, vorzugsweise physiologisch akzeptable, weichmachende Hilfsstoffe zugesetzt werden, um deren Glasübergangstemperatur herabzusetzen. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Die vorstehend beschriebenen erfindungsgemäß geeigneten Polymerisate und ggf. zur Anwendung kommenden Hilfsstoffe werden erfindungsgemäß unter dem Begriff Bindemittel zusammengefasst. Ein erfindungsgemäß geeignetes Bindemittel umfasst also wenigstens ein Bindemittel der oben beschriebenen Art und ggf. ein oder mehrere der oben genannten Hilfsstoffe.

Übliche Additive, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50° C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- oder Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht; Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidantien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel sowie Tenside zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Halv. 61, 69-88 (1986) angegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter Wirkstoffen im Sinne der Erfindung sind alle Stoffe mit einer physiologischen Wirkung und möglichst geringen Nebenwirkungen zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, und Futter- und Nahrungsmittelwirkstoffe. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So Liegt die Wirkstoffkonzentration im Bereich von 0,1 bis 50, vorzugsweise mindestens 20, insbesondere 30 Gew.-% (bezogen auf das Gesamtgewicht der Dosierungsform). Auch Wirkstoff-Kombinationen können eingesetzt werden. Wirkstoffe im Sinne der Erfindung sind auch Vitamine und Mineralstoffe, wie sie auch in Nahrungs- und Futtermitteln enthalten sein können, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin-B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I-und J-Gruppe, K-Gruppe und P-Gruppe. Zu Wirkstoffen im Sinne der Erfindung gehören auch Peptidtherapeutika.

Das erfindungsgemäße Verfahren ist beispielsweise zur Verarbeitung folgender Wirkstoffe geeignet:
Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxocyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrit, Isotretinoin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxyzol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin, Zotepin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Im Einzelnen kann es zur Ausbildung von festen Lösungen oder Dispersionen kommen. Die Begriffe "feste Lösungen" und "feste Dispersionen" sind dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglycol, Glycerin und Polyethylenglycole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Im Allgemeinen sind die Komponenten in dem Lösungsmittel in möglichst hoher Konzentration enthalten. Die Menge richtet sich nach dem zur Anwendung kommenden Lösungsmittel und der Komponente.

Erfindungsgemäß kann man alle Komponenten zu einer Vormischung zusammengeben oder einzelne Komponenten, zumindest in Teilen zu einem späteren Zeitpunkt, beispielsweise während des Extrudierens, zufügen. Es empfiehlt sich beispielsweise, empfindliche Wirkstoffe oder reaktive Komponenten erst zu einem relativ späten Verfahrenszeitpunkt zuzumischen, um diese möglichst kurze Zeit erhöhten Scherkräften und/oder Temperaturen beim Mischen und/oder Extrudieren auszusetzen.

Als Mischapparat sind solche Vorrichtungen brauchbar, die auch in der Kunststofftechnologie zum Mischen eingesetzt werden. Geeignete Vorrichtungen sind beispielsweise beschrieben in "Mischen beim Herstellen und Verarbeiten von Kunststoffen", H.Pahl, VdI-Verlag 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP. DTP der Firma List und Reaktotherm der Firma Krauss-Maffei), Doppelmuldenkneter (Trogmischer) und Stempelkneter (Innenmischer) oder Rotor/Stator-Systeme (z. B. Dispax der Firma Ika).

Das Beschicken der Mischvorrichtung kann kontinuierlich oder diskontinuierlich in üblicher Weise erfolgen. Pulverförmige Komponenten können in freiem Zulauf, beispielsweise über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Die Verfahrensschritte Vermischen und Extrudieren können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt in den Extruder eingespeist und anschließend ggf. unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Da bei der Extrusion ein Lösungsmittel verdampft werden muss, sind die Extruder im Allgemeinen mit einem verdampfungsteil ausgerüstet. Besonders bevorzugt sind Extruder der ZKS-Baureihe von Werner u. Pfleiderer.

Da man erfindungsgemäß zumindest einen Teil der Komponenten in flüssiger Form einsetzt, wird eine Wasser-enthaltende Masse extrudiert. Stellt man beispielsweise eine Vormischung her, die wenigstens eine der Komponenten in flüssiger Form umfasst, kann diese flüssigkeitshaltige Masse beispielsweise in einen Extruder eingespeist und anschließend ausgeformt werden. Dabei ist es natürlich auch möglich, zumindest einen Teil des Wirkstoffes, ggf. auch als Dispersion oder Lösung, erst während des Extrudierens, d.h. im Extruder zuzudosieren.

Eine weitere Alternative besteht darin, eine Komponente, z. B. ein Teil des Bindemittels, ggf. im Gemisch mit wenigstens einem Teil des Wirkstoffes und/oder wenigstens einem Teil der Additive, in Form einer Schmelze einzusetzen, wobei eine solche Schmelze erhalten werden kann, indem man beispielsweise ein lösungsmittelfreies Bindemittel aufschmilzt. Die ggf. im Gemisch vorhandenen Wirkstoff- und/oder Additivanteile können vor, während oder nach dem Aufschmelzen zugefügt werden. Vorzugsweise erfolgt das Aufschmelzen in dem Extruder, mit dem extrudiert wird.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Nach dem Austrag aus dem Extruder erfolgt in der Regel eine abschließende Formgebung. Dabei kann eine Vielzahl von Formen, je nach Extrusionswerkzeug und Art der Formung, erzeugt werden. Beispielsweise lässt sich der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß- oder Kaltabschlag des Stranges können weitere Formen erhalten werden, beispielsweise kleinteilige und gleichmäßig geformte Granulate. Die Heißgranulierung führt in der Regel zu linsenförmigen Tabletten mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Arzneiformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Diese Arzneiformen können in einem nachgeschalteten Verfahrensschritt nach üblichen Methoden gerundet und/oder mit einem Coating versehen werden.

Das erfindungsgemäße Verfahren hat den Vorteil, dass die Staubbildung durch Verwendung fester Komponenten unterdrückt und somit die Kontamination der Produktionsanlage und der Reinigungsaufwand verringert werden können. Dies ist insbesondere bei kritischen Wirkstoffen mit hohem allergenem oder toxischem Potential von Bedeutung.

Ein weiterer Vorteil des Verfahrens besteht darin, dass die Feststoffdosierprobleme durch Verklumpen und Verkleben der Komponenten verringert werden. Außerdem wird die Gefahr des Entmischens der festen Komponenten beseitigt. Ein weiterer Vorteil besteht darin, dass die Komponenten, die kommerziell häufig in flüssiger Form angeboten werden, direkt ohne zusätzlichen Aüfbereitungsschritt eingearbeitet werden können.

Ferner können unerwünschte Reaktionen der Komponenten während der Lagerung in Form einer Pulvermischung vermieden werden.

Schließlich besitzt die Schmelze beim erfindungsgemäßen Verfahren aufgrund des Lösungsmittelgehaltes eine geringere Viskosität. Außerdem muß das Lösungsmittel im Extruder verdampft werden, wobei die Schmelze aufgrund der notwendigen Verdampfungswärme gekühlt wird. Das Verfahren ist daher sehr schonend.

Das nachfolgende Beispiel erläutert die Erfindung.

### Beispiel 1:

In einem Doppelschnecken-Extruder befand sich eine Masse aus einer Mischung von Polyvinylpyrrolidon mit einem K-Wert von 30 und einem Anteil von 20 Gew.% Ibuprofen. In diesen Extruder wurden dann kontinuierlich über eine Dosiervorrichtung etwa 10 ml/min einer 30 Gew.-% Dispersion eines Methacrylsäure/Ethylacrylat (1/1)-copolymers in den kalten Bereich der Förderzone zudosiert. Diese Mischung wurde unter den folgenden Bedingungen extrudiert:

| | |
|---|---|
| Schuss 1 | 60 °C |
| Schuss 2 | 80 °C |
| Schuss 3 | 100 °C |
| Schuss 4 | 110 °C |
| Schuss 5 | 100 °C |
| Schuss 6 | 90 °C |
| Düse | 110 °C |

An Schuss 5 befand sich zusätzlich ein Vakuumvorstoß, so dass das Lösungsmittel abgezogen werden konnte.

Man erhielt eine homogene Masse, die nach Abkühlen gemahlen und zu Tabletten verpresst wurde.

## Patentansprüche

1. Verfahren zur Herstellung fester Dosierungsformen durch Vermischen von 50 bis 99,9 Gew.-% wenigstens eines polymeren Bindemittels, das ausgewählt ist unter Poly(meth)acrylaten, deren Copolymerisaten mit (Meth)acrylsäure, Polyvinyllactamen, Polyvinylestern und Copolymerisaten aus N-Vinyllactamen und Vinylestern, 0,1 bis 50 Gew.-% jeweils bezogen auf das Gesamtgewicht der Dosierungsform wenigstens eines Wirkstoffs und gegebenenfalls üblichen Additiven, Extrudieren des Gemisches, wobei man zumindest einen Teil des polymeren Bindemittels in Form einer wässrigen Dispersion einsetzt und das Dispersionsmittel im Extruder verdampft, und Formgebung.

2. Verfahren nach Anspruch 1, **dadurch gekennzeichnet, dass** das Bindemittel ausgewählt ist unter Poly(meth)acrylaten und deren Copolymerisaten mit (Meth)acrylsäure.

3. Verfahren nach Anspruch 1, **dadurch gekennzeichnet, dass** das Bindemittel ausgewählt ist unter Polyvinyllactamen, insbesondere Polyvinylpyrrolidon, und deren Copolymerisaten mit Vinylestern, insbesondere Vinylacetat.

4. Verfahren nach Anspruch 1, **dadurch gekennzeichnet, dass** man als Bindemitteldispersion eine Methacrylsäure/Ethylacrylat-Dispersion einsetzt.

5. Verfahren nach Anspruch 3, **dadurch gekennzeichnet, dass** man als Bindemittel ein N-Vinylpyrrolidon/Vinylacetat-Copolymer einsetzt.

6. Verfahren nach einem der vorhergehenden Ansprüche, **dadurch gekennzeichnet, dass** man den Wirkstoff in Form einer wässrigen Lösung oder in Form einer Lösung in einem organischen Lösungsmittel einsetzt.

7. Verfahren nach Anspruch 6, **dadurch gekennzeichnet, dass** man als organisches Lösungsmittel ein mit Wasser mischbares Lösungsmittel, insbesondere ein C₁-C₄-Alkanol, verwendet.

8. Verfahren nach Anspruch 7, **dadurch gekennzeichnet, dass** man eine Lösung von Ibuprofen, Ketoprofen, Flurbiprofen, Acetylsalicylsäure, Verapamil, Paracetamol, Nifedipin oder Captopril in einem C₁-C₄-Alkanol einsetzt.

9. Verfahren nach einem der Ansprüche 2 bis 8, **dadurch gekennzeichnet, dass** man eine Dispersion des polymeren Bindemittels einsetzt, die den Wirkstoff gelöst oder dispergiert enthält.

10. Verfahren nach einem der vorhergehenden Ansprüche, **dadurch gekennzeichnet, dass** man aus allen Komponenten eine Vormischung herstellt und diese extrudiert.

11. Verfahren nach einem der Ansprüche 1 bis 8, **dadurch gekennzeichnet, dass** man wenigstens einen Teil des Wirkstoffes während des Extrusionsvorganges zumischt.

12. Verfahren nach Anspruch 1, **dadurch gekennzeichnet, dass** man einen Teil des Bindemittels, gegebenenfalls im Gemisch mit wenigstens einem Teil des Wirkstoffes und/oder wenigstens einem Teil der Additive, in Form einer Polymerschmelze einsetzt.

13. Verfahren nach einem der Ansprüche 1 bis 5, **dadurch gekennzeichnet, dass** man das Bindemittel, gegebenenfalls im Gemisch mit wenigstens einem Teil des Wirkstoffes und/oder wenigstens einem Teil der Additive, in Form von wenigstens zwei Dispersionen oder wenigstens einer Dispersion und einer Lösung einsetzt.

14. Verfahren nach Anspruch 1, **dadurch gekennzeichnet, dass** man das Gemisch durch Koextrusion zu einer mehrschichtigen pharmazeutischen Form extrudiert.

## Claims

1. A process for producing solid dose forms by mixing 50 to 99.9% by weight of at least one polymeric binder, selected from poly(meth)acrylates, their copolymers with (meth)acrylic acid, polyvinyllactams, polyvinyl esters and copolymers of N-vinyllactams and vinyl esters with 0.1 to 50% by weight, in each case based on the overall weight of the dose form, of at least one active ingredient and with or without conventional additives, extruding the mixture, at least part of the polymeric binder being employed in the form of an aqueous dispersion and the dispersion medium being evaporated in the extruder, and shaping.

2. A process as claimed in claim 1, wherein the binder is selected from poly(meth)acrylates and copolymers thereof with (meth)acrylic acid.

3. A process as claimed in claim 1, wherein the binder is selected from polyvinyllactams, especially polyvinylpyrrolidone, and copolymers thereof with vinyl esters, especially vinyl acetate.

4. A process as claimed in claim 1, wherein a methacrylic acid/ethyl acrylate dispersion is employed as binder dispersion.

5. A process as claimed in claim 3, wherein a N-vinylpyrrolidone/vinyl acetate copolymer is employed as binder.

6. A process as claimed in any of the preceding claims, wherein the active ingredient is employed in the form of an aqueous solution or in the form of a solution in an organic solvent.

7. A process as claimed in claim 6, wherein a water-miscible solvent, especially a C₁-C₄-alkanol, is used as organic solvent.

8. A process as claimed in claim 7, wherein a solution of ibuprofen, ketoprofen, flurbiprofen, acetylsalicylic acid, verapamil, paracetamol, nifedipirie or captopril in a C₁-C₄-alkanol is employed.

9. A process as claimed in any of claims 2 to 8, wherein a dispersion of the polymeric binder is employed which contains the active ingredient in solution or in dispersion.

10. A process as claimed in any of the preceding claims, wherein a premix of all the components is prepared and this premix is extruded.

11. A process as claimed in any of claims 1 to 8, wherein at least part of the active ingredient is admixed during the extrusion process.

12. A process as claimed in claim 1, wherein part of the binder, alone or as a mixture with at least part of the active ingredient and/or some of the additives, is employed in the form of a polymer melt.

13. A process as claimed in any of claims 1 to 5, wherein the binder, alone or as a mixture with at least part of the active ingredient and/or at least some of the additives, is employed in the form of at least two dispersions or at least one dispersion and one solution.

14. A process as claimed in claim 1, wherein the mixture is coextruded to form a multilayer pharmaceutical form.

## Revendications

1. Procédé pour la préparation de formes de dosage solides par mélange de 50 à 99,9 % en poids d'au moins un liant polymère choisi parmi les poly(méth)acrylates, les copolymères de (méth)acrylates et d'acide (méth)acrylique, les polyvinyllactames, les esters polyvinyliques et les copolymères de N-vinyllactames et d'esters vinyliques, 0,1 à 50 % en poids, dans les deux cas par rapport au poids total de la forme de dosage, d'au moins une substance active et le cas échéant d'additifs usuels, extrusion du mélange, une partie au moins du liant polymère étant mise en oeuvre sous forme d'une dispersion aqueuse et le milieu de dispersion vaporisé dans l'extrudeuse, et formage.

2. Procédé selon la revendication 1, **caractérisé par le fait que** le liant est choisi parmi les poly(méth)acrylates et les copolymères de (méth)acrylates et d'acide (méth)acrylique

3. Procédé selon la revendication 1, **caractérisé par le fait que** le liant est choisi parmi les polyvinyllactames, plus spécialement la polyvinylpyrrolidone, et les copolymères de vinyllactames et d'esters vinyliques, en particulier de l'acétate de vinyle.

4. Procédé selon la revendication 1, **caractérisé par le fait que** l'on met en oeuvre une dispersion de liant consistant en une dispersion d'acide méthacrylique/acrylate d'éthyle.

5. Procédé selon la revendication 3, **caractérisé par le fait que** le liant utilisé est un copolymère N-vinylpyrrolidone/acétate de vinyle.

6. Procédé selon l'une des revendications qui précèdent, **caractérisé par le fait que** la substance active est mise en oeuvre à l'état de solution aqueuse ou à l'état de solution dans un solvant organique.

7. Procédé selon la revendication 6, **caractérisé par le fait que** le solvant organique utilisé est un solvant miscible à l'eau, plus spécialement un alcanol en C1-C4.

8. Procédé selon la revendication 7, **caractérisé par le fait que** l'on met en oeuvre une solution d'Ibuprofen, de Ketoprofen, de Flurbiprofen, d'acide acétylsalicylique, de Verapamil, de Paracetamol, de Nifedipin ou de Captopril dans un alcanol en C1-C4.

9. Procédé selon l'une des revendications 2 à 8, **caractérisé par le fait que** l'on met en oeuvre une dispersion du liant polymère contenant la substance active à l'état dissous ou dispersé.

10. Procédé selon l'une des revendications qui précèdent, **caractérisé par le fait que** l'on forme un mélange préalable à partir de tous les composants et on extrude ce mélange.

11. Procédé selon l'une des revendications 1 à 8, **caractérisé par le fait que** l'on ajoute et on mélange une partie au moins de la substance active au cours de l'opération d'extrusion.

12. Procédé selon la revendication 1, **caractérisé par le fait que** l'on met en oeuvre une partie du liant, le cas échéant en mélange avec au moins une partie de la substance active et/ou au moins une partie des additifs, à l'état de masse polymère fondue.

13. Procédé selon l'une des revendications 1 à 5, **caractérisé par le fait que** l'on met en oeuvre le liant, éventuellement en mélange avec au moins une partie de la substance active et/ou au moins une partie des additifs, sous forme d'au moins deux dispersions ou d'au moins une dispersion une solution.

14. Procédé selon la revendication 1, **caractérisé par le fait que** le mélange est mis à l'état de forme pharmaceutique à couches multiples par co-extrusion.
